# EUROPÄISCHE PATENTSCHRIFT

(11) **EP 0 546 409 B1**
(45) Veröffentlichungstag und Bekanntmachung des Hinweises auf die Patenterteilung: **21.10.1998**
(21) Anmeldenummer: 92120405.3
(22) Anmeldetag: 30.11.1992
(51) Int. Cl.: C07D 309/08

(54) **Verfahren zur Herstellung von Tetrahydropyrancarbonsäureestern**
Method for the preparation of tetrahydropyrancarboxylic esters
Méthode pour la préparation des esters tétrahydropyranecarboxyliques

(30) Priorität: 13.12.1991 DE 4141219
(43) Veröffentlichungstag der Anmeldung: 16.06.1993
(73) Patentinhaber: BASF Aktiengesellschaft, 67063 Ludwigshafen (DE)
(72) Erfinder: Kuekenhoehner, Thomas, Dr., W-6737 Boehl-Iggelheim (DE); Goetz, Norbert, Dr., W-6520 Worms 1 (DE); Fischer, Rolf, Dr., W-6900 Heidelberg (DE); Schnurr, Werner, Dr., W-6719 Herxheim (DE)

(56) Entgegenhaltungen:
- JOURNAL OF THE CHEMICAL SOCIETY, 1952, Letchworth,GB, Seiten 2268 - 2272 C.T. BLOOD, ET AL.: 'The preparation of 4,4'-bistetrahydropyranyl and of ethane-1,1,2,2-tetraacetic acid'

## Beschreibung

Die Erfindung betrifft ein Verfahren zur Herstellung von Tetrahydropyrancarbonsäureestern durch Umsetzung von Malonsäureestern mit β,β'-difunktionalisierten Diethylethern in Gegenwart von Alkoholaten und einem dipolar aprotischen Lösungsmittel.

Aus J. Chem. Soc. (1930), Seite 2525 bis 2530 ist die Herstellung beispielsweise von Tetrahydropyran-4,4-dicarbonsäure-diethylester durch Reaktion von β,β'-Dichlordiethylether mit Malonsäurediethylester und Natriumethylat in Ethanol mit 40%iger Ausbeute bekannt. Nach einer verbesserten Verfahrensvariante läßt sich Tetrahydropyran-4,4-dicarbonsäurediethylester mit einer Ausbeute von 63 % erhalten (J. Chem. Soc. 1952, Seite 2268).

Die bisher bekannten verfahren lassen z.B. in ihren Ausbeuten zu wünschen übrig.

Es bestand daher die Aufgabe, ein Verfahren zu entwickeln, das den zuvor genannten Nachteilen abhilft.

Demgemäß wurde ein neues und verbessertes Verfahren zur Herstellung von Tetrahydropyrancarbonsäureestern der allgemeinen Formel I 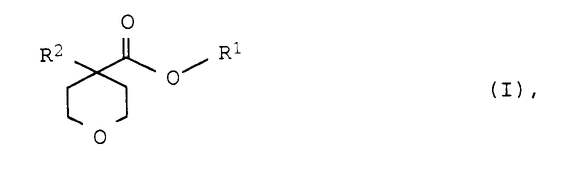 in der
- R¹: Methyl oder Ethyl und
- R²: die Gruppe COOR¹
bedeuten, durch Umsetzung von 1,3-Dicarbonylverbindungen der allgemeinen Formel II 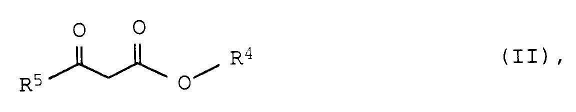 in der
- R⁴: Methyl oder Ethyl und
- R⁵: die Gruppe OR⁴
bedeutet, mit Ethern der allgemeinen Formel III 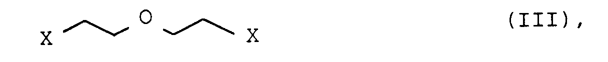 in der
- X: für Chlor, Brom oder eine der Gruppen OCOCl oder OSOCl
steht, in Gegenwart von Alkoholaten, gefunden, welches dadurch gekennzeichnet ist, daß man die Umsetzung in Gegenwart eines dipolar aprotischen Lösungsmittels durchführt.

Mit dem erfindungsgemäßen Verfahren gelingt die Darstellung der Tetrahydropyrandicarbonsäureester I in überraschend hohen Ausbeuten von über 70 % bei Verwendung von Malonsäureestern und den bifunktionellen Etherderivaten III als Ausgangsmaterialien, wenn in Gegenwart von dipolar aprotischen Lösungsmitteln gearbeitet wird:

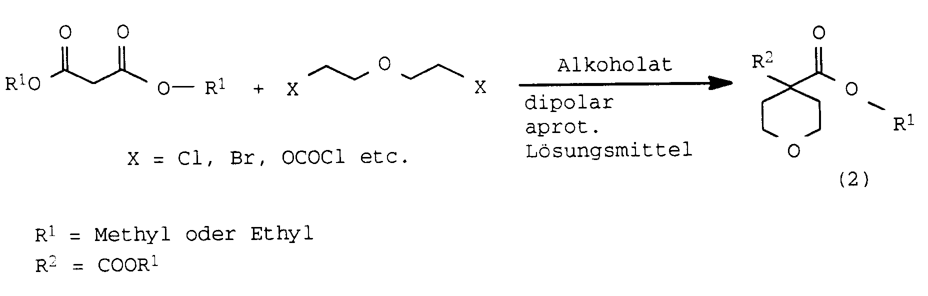

Das erfindungsgemäße Verfahren kann wie folgt durchgeführt werden:

In der Regel legt man die 1,3-Dicarbonylverbindung II und den Ether III in einem dipolaren aprotischen Lösungsmittel vor. Bei Temperaturen von 40 bis 180°C, bevorzugt 60 bis 160°C, besonders bevorzugt 90 bis 130°C und Drücken von 0,01 bis 10 bar, bevorzugt 0,1 bis 5 bar, besonders bevorzugt bei 0,3 bis 1 bar (Atmosphärendruck) kann anschließend unter intensivem Rühren das Alkoholat z.B. in Form seiner Alkoholat-Lösung zudosiert werden.

Es kann vorteilhaft sein, in der Reaktionsmischung die Konzentration an Alkohol, der aus dem Alkoholat freigesetzt wird bzw. der gegebenenfalls durch die Alkoholat-Lösung eingebracht wird, möglichst niedrig zu halten. Um dies zu erreichen, kann beispielsweise der Alkohol schon während der Alkoholat-Zudosierung abdestilliert werden. Je nach Reaktionstemperatur kann diese Destillation und damit auch die Reaktion bei Normaldruck, im Vakuum, etwa bis 100 mbar, oder unter leichtem Überdruck, etwa bis 3 bar, erfolgen.

Als dipolare aprotische Lösungsmittel eignen sich Dialkylformamide wie C₃- bis C₃₀-Dialkylformamide, bevorzugt C₃- bis C₂₀-Dialkylformamide z.B. Dimethylformamid, Diethylformamid, Di-n-propylformamid, Di-isopropylformamid, Di-n-butylformamid, Di-iso-butylformamid, Di-sec.-butylformamid, Di-tert.-butylformamid, Methyl-ethylformamid, Methyl-n-propylformamid, Methyl-isopropyl-formamid, Methyl-n-butylformamid, Methyl-isobutyl-formamid, Methyl-sec.-butyl-formamid, Methyl-tert.-butyl-formamid, Ethyl-n-propyl-formamid, Ethylisopropyl-formamid, Ethyl-n-butyl-formamid, Ethyl-iso-butylformamid, Ethyl-sec.-butyl-formamid, Ethyl-tert.-butyl-formamid, N-Formylpyrrolidin, N-Formylpiperidin und N-Formylmorpholin,

Dialkylacetamide wie C₄- bis C₂₀-Dialkylacetamide, bevorzugt C₄- bis C₁₀-Dialkylacetamide z.B. Dimethylacetamid, Diethylacetamid, Di-n-propylacetamid, Di-iso-propylacetamid, Di-n-butylacetamid, Di-iso-butylacetamid, Di-sec.-butylacetamid, Di-tert.-butylacetamid, Methyl-ethyl-acetamid, Methyl-n-propyl-acetamid, Methyl-iso-propyl-acetamid, Methyl-n-butyl-acetamid, Methyl-iso-butyl-acetamid, Methyl-sec.-butyl-acetamid, Methyl-tert.-butyl-acetamid, Ethyl-n-propyl-acetamid, Ethyl-iso-propyl-acetamid, Ethyl-n-butyl-acetamid, Ethyl-iso-butyl-acetamid, Ethyl-sec.-butyl-acetamid, Ethyl-tert.-butyl-acetamid, N-Acetylpyrrolidin, N-Acetylpiperidin und N-Acetylmorpholin,

Dialkylsulfoxide wie C₂- bis C₂₀-Dialkylsulfoxide, bevorzugt C₃- bis C₁₀-Dialkylsulfoxide z.B. Dimethylsulfoxid, Diethylsulfoxid, Di-n-propylsulfoxid, Di-iso-propylsulfoxid, Di-n-butylsulfoxid, Di-iso-butylsulfoxid, Di-sec.-butylsulfoxid, Di-tert.-butylsulfoxid, Methyl-ethyl-sulfoxid, Methyl-n-propyl-sulfoxid, Methyl-iso-propyl-sulfoxid, Methyl-n-butyl-sulfoxid, Methyl-iso-butyl-sulfoxid, Methyl-sec.-butyl-sulfoxid, Methyl-tert.-butyl-sulfoxid, Ethyl-n-propyl-sulfoxid, Ethyl-iso-propyl-sulfoxid, Ethyl-n-butyl-sulfoxid, Ethyl-iso-butyl-sulfoxid, Ethyl-sec.-butyl-sulfoxid, Ethyl-tert.-butyl-sulfoxid, cyclische Sulfoxide wie z.B. Tetramethylensulfoxid,

Dialkylsulfone wie C₂- bis C₂₀-Dialkylsulfone, Dialkylsulfone, bevorzugt C₃- bis C₁₀-Dialkylsulfone z.B. Dimethylsulfon, Diethylsulfon, Di-n-propylsulfon, Di-iso-propylsulfon, Di-n-butylsulfon, Di-iso-butylsulfon, Di-sec.-butylsulfon, Di-tert.-butylsulfon, Methyl-ethyl-sulfon, Methyl-n-propyl-sulfon, Methyl-iso-propyl-sulfon, Methyl-n-butyl-sulfon, Methyl-iso-butyl-sulfon, Methyl- sec.-butyl-sulfon, Methyl-tert.-butyl-sulfon, Ethyl-n-propyl-sulfon, Ethyl-iso-propyl-sulfon, Ethyl-n-butyl-sulfon, Ethyl-iso-butyl-sulfon, Ethyl-sec.-butyl-sulfon, Ethyl-tert.-butyl-sulfon, cyclische Sulfone wie z.B. Tetramethylensulfon,

N-Alkylpyrrolidone wie N-Methylpyrrolidon, N-Ethylpyrrolidon, N-n-Propylpyrrolidon, N-iso-Propylpyrrolidon, N-n-Butylpyrrolidon, N-iso-Butylpyrrolidon, N-sec.-Butylpyrrolidon und N-tert.-Butylpyrrolidon,

N,N'-Dialkylpropylenharnstoffe wie N,N'-Dimethylpropylenharnstoff, Dimethylpropylenharnstoff, Diethylpropylenharnstoff, Dipropylpropylenharnstoff und Diisopropylpropylenharnstoff und deren Gemische,

N,N'-Dialkylethylenharnstoffe wie N,N'-Dimethylethylenharnstoff, Diethylethylenharnstoff, Dimethylethylenharnstoff, Dipropylethylenharnstoff und Diisopropylethylenharnstoff und deren Gemische.

Verwendung können ebenfalls Mischungen dieser Lösungsmittel finden.

Das Molverhältnis von 1,3-Dicarbonylverbindungen II zum Ether III beträgt in der Regel 0,2 : 1 bis 5 : 1, bevorzugt 0,75 : 1 bis 1,5 : 1, besonders bevorzugt 0,9 : 1 bis 1,1 : 1 (etwa äquimolar).

Die Menge des verwendeten dipolar-aprotischen Lösungsmittels kann in breiten Bereichen von etwa 100 g je eingesetztem Mol der Dicarbonylverbindung II bis ca. 3000 g/Mol variieren. Bevorzugt werden 200 g/Mol bis 2000 g/Mol, besonders bevorzugt 300 g/Mol bis 1000 g/Mol eingesetzt.

Als 1,3-Dicarbonylverbindungen II eignen sich Malonsäureester beliebiger Alkohole. In der Regel bringt jedoch die Verwendung von Estern höherer Alkohole als C₂ keine nennenswerten Vorteile, so daß üblicherweise die Methyl- oder Ethylester, verwendet werden. Besondere bevorzugt sind die Methylester.

Als Ether III eignen sich beispielsweise β,β'-Dichlordiethylether, β,β'-Dibromdiethylether, Diglykolbischlorformiat oder der Bischloroschwefligsäureester von Diethylenglycol, der z.B. in situ aus Diglykol und Thionylchlorid hergestellt und der leicht in situ zu β,β'-Dichlordiethylether umgewandelt werden kann. Bevorzugt sind β,β'-Dichlordiethylether und Diglykolbischlorformiat.

Die Malonester II sind entweder bekannt oder können nach allgemeinen bekannten Verfahren hergestellt werden. Die als Ausgangsmaterialien besonders bevorzugten Acetessigsäure- bzw. Malonsäure-methyl- oder Ethylester sind großtechnische Produkte. Die Ether III sind ebenfalls bekannt und z.T. großtechnisch verfügbar.

Als Alkoholate eignen sich z.B. Erdalkali- und Alkalimetallalkoholate wie Magnesium-, Calcium-, Lithium-, Natrium- und Kaliumalkoholate beliebiger Alkohole. Üblicherweise werden die Alkalimetallalkoholate von C₁- bis C₄-Alkoholen verwendet, da die Verwendung von Alkalimetallalkoholaten höherer Alkohole in der Regel keine nennenswerten Vorteile bringt. Besonders bevorzugte Alkalimetallalkoholate sind z.B. Natriumethylat, Kaliummethylat, Kaliumethylat, Kalium-tert.-butylat und insbesondere Natriummethylat.

Die Alkoholate können als Feststoff in die Reaktion eingesetzt werden. Aus verfahrenstechnischen Gründen ist es jedoch vorteilhaft, mit Lösungen der Alkoholate zu arbeiten. Verwendung finden können im Prinzip alle Lösungsmittel, die in Gegenwart der verwendeten Alkoholate und unter den Reaktionsbedingungen stabil sind. Besonders vorteilhaft sind alkoholische Lösung der Alkoholate, da diese Lösungen einfach hergestellt werden können, z.B. durch Auflösen der jeweiligen Alkalimetalle in dem betreffenden Alkohol. Besonders bevorzugt wird die Lösung von Natriummethylat in Methanol.

Werden unterschiedliche Alkohole bzw. Alkoholreste bei der verwendeten 1,3-Dicarbonylverbindung II und dem Alkoholat bzw. der Alkoholatlösung verwendet, so finden unter den Reaktionsbedingungen üblicherweise Umesterungsreaktionen statt. Auf diese Weise können bei Verwendung von Malonsäureestern auch Tetrahydropyrandicarbonsäureester I mit unterschiedlichen Estergruppen hergestellt werden. Wird durch Abdestillation während der Reaktion der niedrigsiedende Alkohol entfernt, so läßt sich die in-situ-Umesterung dazu nutzen, praktisch vollständig den Tetrahydropyranester oder -diester mit der höher siedenden Alkoholkomponente herzustellen. Gegebenenfalls kann es in solchen Fällen auch vorteilhaft sein, die im Produkt gewünschte Alkoholkomponente dem Reaktionsgemisch zuzugeben.

In einigen Fällen, insbesondere wenn die verwendeten Ausgangssubstanzen eine geringe Reaktivität besitzten, kann es vorteilhaft sein, die Reaktion in Gegenwart von katalytischen Mengen Bromid- oder Iodidsalzen durchzuführen. Dadurch werden in einigen Fällen nicht nur eine schnellere Reaktion, sondern auch höhere Ausbeuten der Tetrahydropyranester I erzielt. Die Katalysatormenge kann dabei in weiten Bereichen gewählt werden. Üblicherweise empfehlen sich Mengen von 0,01 bis 20 Mol-%, bevorzugt 1 bis 10 Mol-%, bezogen auf die eingesetzte 1,3-Dicarbonylverbindung. Höhere Mengen sind ebenfalls möglich, bringen jedoch in aller Regel keine nennenswerten Vorteile.

Wird für die in situ-Herstellung z.B. von β,β'-Dichlordiethylether das Diglykolbischlorformiat verwendet, so können für die Zersetzung des Formiates katalytische Mengen quartärer Ammoniumsalze oder Salze von tertiären Aminen verwendet werden. Geeignete quartäre Ammoniumsalze sind beispielsweise Triethylbenzylammoniumchlorid, Trimethylbenzylammoniumchlorid, Trimethylbenzylammoniumbromid, Triphenylbenzylammoniumchlorid, Methyltributylammoniumiodid, Tetrabutylammoniumchlorid, Tetrabutylanmoniumbromid, Methyltrioctylammoniumchlorid oder Salzmischungen, wie sie z.B. unter der Bezeichnung Aliquat® im Handel erhältlich sind. Als Salze tertiärer Amine sind im Prinzip die Hydrochloride oder Hydrobromide aller Amine mit inerten Resten, insbesondere Alkylresten, geeignet, für die Praxis empfiehlt sich jedoch die Verwendung solcher Amine, deren Reste 3 bis 12 C-Atome tragen. Besonders gut geeignet sind die Hydrochloride von Tri-n-propylamin und von Tri-n-butylamin.

Bei der Zersetzung des Diglykolbischlorformiates kann im Prinzip jedes inerte Verdünnungsmittel zugesetzt werden, üblicherweise bringt dies aber keine nennenswerten Vorteile, so daß die Zersetzung in der Praxis ohne Verdünnungsmittel durchgeführt wird.

Die Zersetzung des Diglykolbischlorformiates kann in einem weiten Temperaturbereich vorgenommen werden. Üblicherweise liegt sie zwischen 90 und 170°C, bevorzugt wird die Temperatur in einem Bereich zwischen 100 und 130°C gewählt.

Die Zersetzung von Chlorformiaten ist allgemein in der EP-A-25 829 beschrieben.

Das Verfahren läßt sich mit den üblichen Verfahrenstechniken - Einsatz von Rohrreaktoren oder Rührkesselkaskaden - auch kontinuierlich betreiben. Die Aufarbeitung des Reaktionsgemisches kann nach üblichen Verfahren wie z.B. Destillation oder durch Absaugen der ausgefallenen Salze mit anschließender destillativer Isolierung der Produkte erfolgen. Da sich die Tetrahydropyrancarbonsäureester I im allgemeinen vorzugsweise in organischen Lösungsmitteln lösen, kann die Aufarbeitung des Reaktionsgemisches und die Isolierung der Tetrahydropyrancarbonsäureester I auch auf herkömmliche Weise extraktiv, gegebenenfalls mit anschließender Destillation, erfolgen.

Die Substituenten R¹, R², R⁴, R⁵ und X in den Verbindungen I, II und III haben folgende Bedeutungen:
- R¹, R⁴: - Methyl und Ethyl, bevorzugt Methyl,
- R²: - die Gruppe COOR¹,
- R⁵: - die Gruppe OR⁴,
- X: - Chlor und Brom, bevorzugt Chlor,
- die Gruppe OCOCl,
- die Gruppe OSOCl.

Die nach dem erfindungsgemäßen Verfahren erhältlichen Tetrahydropyrandicarbonsäureester I sind Zwischenprodukte zur Herstellung von Pharmazeutika und insbesondere von Pflanzenschutzmitteln, wie sie z.B. in EP-A-142 741 beschrieben sind.

### Beispiele

### Beispiel 1

In einer Rührapparatur werden 10,5 g Tetraethylammoniumbromid auf 100°C erhitzt. Bei dieser Temperatur werden 1039,5 g Diglykolbischlorformiat in dem Maße zugefügt, daß die einsetzende Kohlendioxidentwicklung nicht zu heftig wird. Nach beendeter Gasentwicklung werden der Reaktionsmischung nacheinander 1400 g Dimethylformamid, 594 g Malonsäuredimethylester und 1620 g 30 gew.%ige methanolische Natriummethylatlösung zugefügt. Die Natriummethylatzugabe erfolgt so, daß die erste Hälfte bei 95 bis 100°C und die zweite Hälfte bei 125 bis 130°C stattfindet. Methanol wird kontinuierlich zunächst bei 530 mbar Unterdruck, später bei Normaldruck abdestilliert. Nach 4 Stunden Reaktionsdauer läßt man auf Raumtemperatur abkühlen und arbeitet auf. Hierzu wird das aus der Lösung ausgefallene Natriumchlorid abfiltriert und das Filtrat fraktioniert. Durch Fraktionierung erhält man 49 g (7,6 %) Tetrahydropyran-4-carbonsäuremethylester und 604,4 g (66 %) Tetrahydropyran-4,4-dicarbonsäuredimethylester (Kp.: 150°C/40 mbar).
¹H-NMR Tetrahydropyran-4,4-dicarbonsäuredimethylester (CDCl₃): δ/ppm = 2,0-2,2 (m,4H); 3,6-3,7 (m,4H); 3,7 (s,6H).

### Beispiel 2

In einer Rührapparatur werden 644 g (4,5 Mol) β,β'-Dichlordiethylether, 594 g (4,5 Mol) Malonsäuredimethylester und 1400 g Dimethylformamid vorgelegt. Die Mischung wird auf 110°C erhitzt, anschließend werden 1620 g einer 30 %igen Lösung von Natriummethylat in Methanol (9 Mol) innerhalb von 4 Stunden zugetropft. Methanol wird während der Natriummethylatzugabe bei 700 mbar kontinuierlich abdestilliert. Nach beendeter Reaktion wird der größte Teil des Lösungsmittels im Vakuum abdestilliert, anschließend werden 895 g Toluol, 1700 g Wasser und 100 g 38 %ige Salzsäure zugegeben; der pH-Wert der wäßrigen Phase stellt sich dabei auf etwa 7 ein. Die wäßrige Phase wird abgetrennt und dreimal mit je 100 ml Toluol nachextrahiert. Die toluolischen Phasen werden vereinigt und anschließend fraktioniert. Durch Fraktionierung werden 22,5 g (3,5 %) Tetrahydro-pyran-4-carbonsäuremethylester und 610,2 g (67 %) Tetrahydropyran-4,4-dicarbonsäuredimethylester (Kp.: 90°C/2 mbar) erhalten.

### Beispiel 3

In einer Rührapparatur werden 5,25 g (0,025 Mol) Tetraethylammoniumbromid auf 100°C erhitzt. Bei dieser Temperatur werden 520 g (2,25 Mol) Diglykolbischlorformiat in dem Maße zugefügt, daß die einsetzende Kohlendioxidentwicklung nicht zu heftig wird. Nach beendeter Gasentwicklung werden der Reaktionsmischung nacheinander 1400 g Dimethylformamid, 297 g (2,25 Mol) Malonsäuredimethylester und 812 g 30 gew.%ige methanolische Natriummethylatlösung zugefügt.

Die Natriummethylatzugabe erfolgt bei 110°C. Methanol wird kontinuierlich bei 570 mbar Unterdruck abdestilliert. Nach 4 Stunden Reaktionsdauer läßt man auf Raumtemperatur abkühlen und arbeitet auf. Hierzu wird zunächst der größte Teil des Lösungsmittels bei 60 mbar abdestilliert, anschließend werden 1000 ml Toluol, 1000 ml Wasser und 25 ml 38 %ige Salzsäure zugegeben. Die wäßrige Phase wird abgetrennt und zweimal mit je 100 ml Toluol nachextrahiert. Die toluolischen Phasen werden vereinigt und anschließend fraktioniert. Man erhält 13,4 g (4 %) Tetrahydropyran-4-carbonsäuremethylester und 312,4 g (69 %) Tetrahydropyran-4,4-dicarbonsäuredimethylester (Kp.: 81°C/1 mbar).

## Patentansprüche

1. Verfahren zur Herstellung von Tetrahydropyrancarbonsäureestern der allgemeinen Formel I 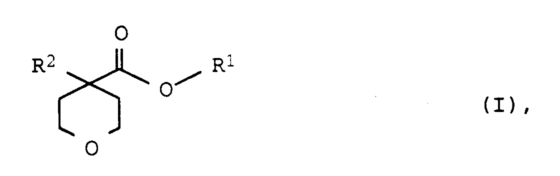 in der
R¹ Methyl oder Ethyl und
R² die Gruppe COOR¹
bedeuten, durch Umsetzung von 1,3-Dicarbonylverbindungen der allgemeinen Formel II 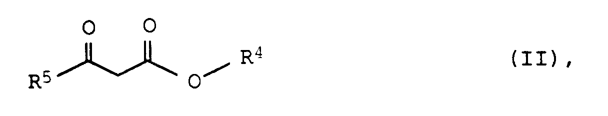
in der
R⁴ Methyl oder Ethyl und
R⁵ die Gruppe OR⁴
bedeutet, mit Ethern der allgemeinen Formel III 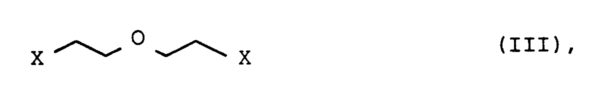
in der
X für Chlor, Brom oder eine der Gruppen OCOCl oder OSOCl
steht, in Gegenwart von Alkoholaten, dadurch gekennzeichnet, daß man die Umsetzung in Gegenwart eines dipolar aprotischen Lösungsmittel durchführt.

2. Verfahren nach Anspruch 1, dadurch gekennzeichnet, daß man die Umsetzung bei Temperaturen von 60 bis 160°C und Drücken von 0,1 bis 5 bar durchführt.

## Claims

1. A process for preparing tetrahydropyrancarboxylic esters of the formula I 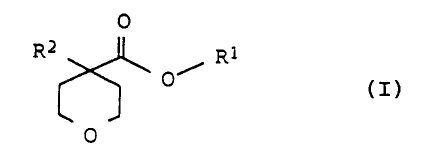 where
R¹ is methyl or ethyl and
R² is COOR¹,
by reacting 1,3-dicarbonyl compounds of the formula II 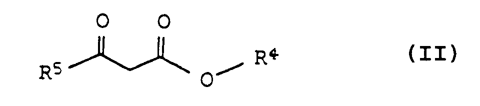 where
R⁴ is methyl or ethyl and
R⁵ is OR⁴,
with ethers of the formula III 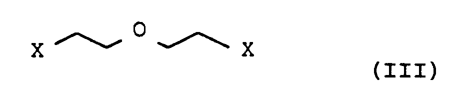 where
X is chlorine, bromine, OCOCl or OSOCl,
in the presence of alcoholates, which reaction is carried out in the presence of a dipolar aprotic solvent.

2. A process as claimed in claim 1, wherein the reaction is carried out at from 60 to 160°C and under from 0.1 to 5 bar.

## Revendications

1. Procédé pour la préparation d'esters d'acides tétrahydropyrannecarboxyliques de formule générale I 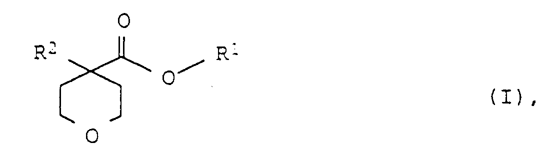 dans laquelle
R¹ représente un groupe méthyle ou éthyle et
R² le groupe COOR¹
par réaction de dérivés 1,3-dicarbonylés de formule générale II 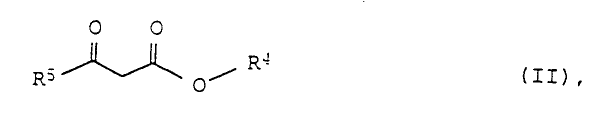 dans laquelle
R⁴ représente un groupe méthyle ou éthyle et
R⁵ le groupe OR⁴,
avec des éthers de formule générale III 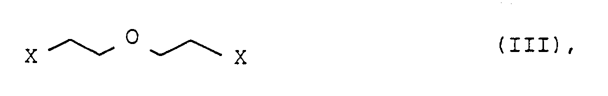 dans laquelle
X représente le chlore, le brome ou l'un des groupes OCOCl ou OSOCl,
en présence d'alcoolates, caractérisé par le fait que la réaction est effectuée en présence d'un solvant aprotonique dipolaire.

2. Procédé selon la revendication 1, caractérisé par le fait que la réaction est effectuée à des températures de 60 à 160°C sous des pressions de 0,1 à 5 bar.
